# EUROPEAN PATENT APPLICATION

(11) **EP 1 484 005 A1**
(43) Date of publication of application: **08.12.2004**
(21) Application number: 04253185.5
(22) Date of filing: 28.05.2004
(51) Int. Cl.: A61B 3/04, A61B 3/028, G02C 7/06

(54) **Improvements in and relating to trial lenses**

(30) Priority: 04.06.2003 GB 0312749
(71) Applicant: Optimed Limited, Coventry, West Midlands CV2 3FJ (GB)
(72) Inventor: Trusit, Dave, Coventry, West Midlands CV2 3FJ (GB)
(74) Representative: Moffat, John Andrew

(57) **Abstract**

The present invention relates to an improved trial lenses and also to the use of such improved trial lenses in instruments used to assess or derive the refraction or refractive status of an individual. It is a disadvantage of known trial lens sets that the lenses in such sets are unifocal, that is single vision lenses. However, this does not allow a practitioner to demonstrate the benefits of bifocals, multifocals or progressives to those who require such lenses when distance refraction lenses are in place over the eyes of a patient. According to the present invention, a lens for use in an instrument used to assess or derive the refraction or refractive status of an individual comprises a distance portion and a near portion.

## Description

The present invention relates to improved trial lenses and also to the use of such improved trial lenses in an instrument used to assess or derive the refraction or refractive status of any given individual.

It is a disadvantage of known trial lens sets that the trial lenses in such sets are unifocal, that is single vision lenses. A trial lens comprises a lens supplied in a standard trial rim, that is a lens holder (see for example BS EN ISO 12867:1998). The term trial lens is used herein to refer to a lens supplied in a standard rim. The trial lenses are adapted to be located in an instrument used to assess or derive the refraction or refractive status of any given individual, such as a trial frame, specifically designed to house trial lenses. For example, a practitioner will place the trial lenses in a trial frame and ask the individual to read from an eye chart. By addition and substitution of the trial lenses a suitable prescription may be derived.

Using this method, distance and near refraction are derived separately.
However, this does not allow a practitioner to demonstrate the benefits of bifocals, multifocals or progressives to those who require such lenses when distance refraction lenses are in place over the eyes of a patient.

There have been a number of proposed solutions to this problem. For example, it is known to align a series of lenses in front of the eyes of an individual by the use of suitably adapted trial frames (as shown for example in US 2 103 798, US 2 835 161 or US 4 988 185). However, each of these proposals requires an adaptation of existing trial frames or new equipment. Such arrangements have not achieved widespread acceptance.

According to the present invention, a lens for use in an instrument used to assess or derive the refraction or refractive status of an individual comprises a distance portion and a near portion.

It is an advantage of the present invention that it provides an eye-care practitioner with the ability to demonstrate the clinical and practical benefit of bifocals and multifocals in a consulting room and optical dispensary with the patient retaining normal head posture.

The invention also has as an advantage that existing equipment used by the practitioner may be used with the present invention. This means that the present invention may be adopted without the need to replace existing instruments used by the practitioner to assess or derive the refraction or refractive status of any given individual.

The distance portion may be formed from a lens of normal (plano) power. The near portion demonstrates the near addition.

According to a second aspect of the present invention, an instrument used to assess or derive the refraction or refractive status of any given individual incorporates at least one lens in accordance with the first aspect of the invention.

Preferably the instrument comprises a trial lens. Preferably, a trial lens set incorporates one or more trial lenses of this kind.

Alternatively the instrument comprises a phoropter.

The bifocal and multifocal trial lenses are used in conjunction with conventional (single vision) trial lenses from a trial lens set or lenses from a phoroptor.

The invention will now be described, by way of example only, with reference to the accompanying drawings, in which:-
Figure 1 shows in diagrammatic form a first embodiment of a trial lens in accordance with the present invention;
Figure 2 shows in diagrammatic form a second embodiment of a trial lens in accordance with the present invention;
Figure 3 shows a trial frame that may be used with the present invention; and
Figure 4 shows a phoropter that may be used with the present invention.

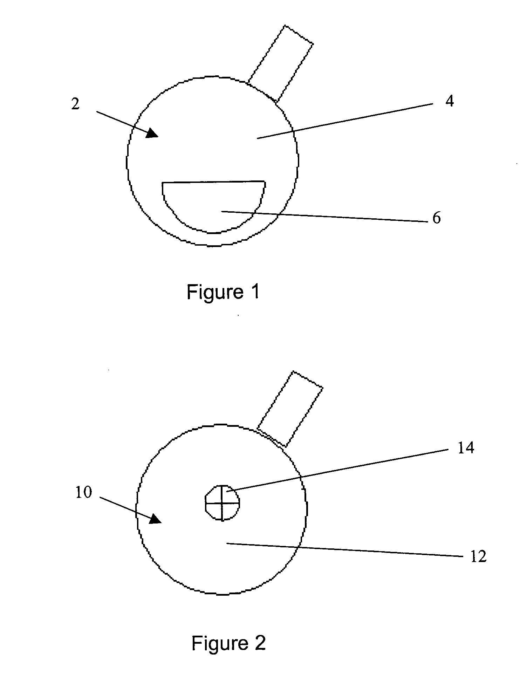

Referring first to Figure 1, there may be seen in diagrammatic form a bifocal trial lens 2 in accordance with the present invention for use in a trial lens set. The lens 2 comprises a first plano portion 4 and a second near addition insert portion 6.

In Figure 2, a progressive lens 10 for use in a trial lens set is seen to comprise a first plano portion 14 and a second multifocal insert portion 12.

An instrument used to assess or derive the refraction or refractive status of any given individual may be used in conjunction with trial lens in accordance with the present invention.

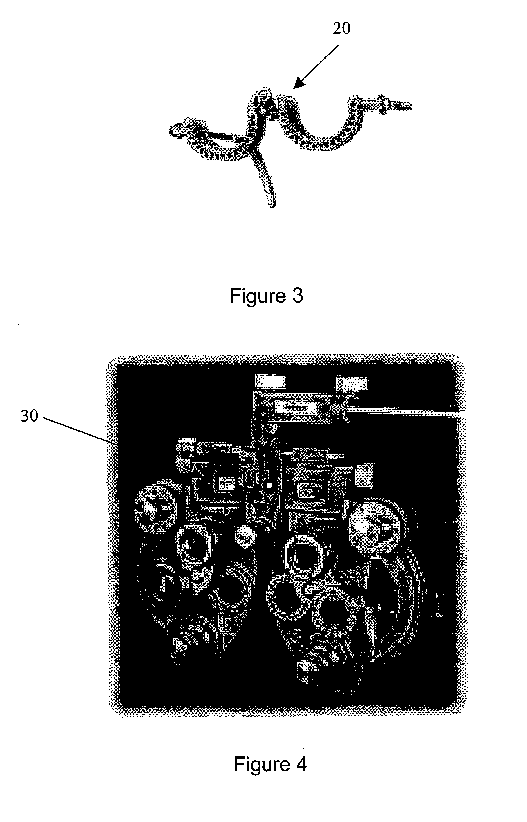

For example, a trial lens rim in accordance with the present invention may be located in a rim of a suitable trial lens set 20 (Figure 3). The rim may be either full aperture or reduced aperture.

Alternatively, one or more trial lens rims may be incorporated in a phoropter 30 (Figure 4).

## Claims

1. A lens for use in an instrument used to assess or derive the refraction or refractive status of an individual comprises a distance portion and a near portion.

2. A lens according to claim 1 in which the distance portion is formed from a lens of normal (plano) power (4,14).

3. A lens according to claim 1 or claim 2 in which the near portion comprises a multifocal insert portion (12) or a progressive insert (6).

4. An instrument used to assess or derive the refraction or refractive status of any given individual incorporating at least one lens in accordance with any of claims 1 to 3.

5. An instrument according to claim 4, in which the instrument comprises a trial lens.

6. A trial lens set incorporating one or more trial lenses in accordance with any of claim 5.

7. An instrument according to claim 4, in which the instrument comprises a phoropter (30).
